# EUROPEAN PATENT APPLICATION

(11) **EP 2 325 330 A1**
(43) Date of publication of application: **25.05.2011**
(21) Application number: 10783302.2
(22) Date of filing: 26.05.2010
(51) Int. Cl.: C12Q 1/68, C12N 15/09

(54) **METHOD FOR SIMULTANEOUS DETECTION OF VIROIDS PSTVd AND TCDVd**

(30) Priority: 02.06.2009 JP 2009133144
(71) Applicant: Incorporated Administrative Agency National Agriculture and Food Research Organization, Ibaraki 305-8517 (JP)
(72) Inventor: MATSUSHITA, Yosuke, Tsukuba-shi Ibaraki 305-8519 (JP); TSUDA, Shinya, Tsukuba-shi Ibaraki 305-8666 (JP)
(74) Representative: Vossius & Partner
(86) International application number: PCT/JP2010/058894
(87) International publication number: WO 2010/140518

(57) **Abstract**

The present invention relates to a method for detecting viroids, comprising: carrying out nucleic acid amplification reaction using RNA from a test plant sample as a template and a primer set comprising (i) a reverse primer of a sequence of 16 to 30 nucleotides in length within the sequence complement of the nucleotide sequence ranging from nucleotide positions 18 to 114 of a DNA sequence corresponding to the PSTVd genome (PSTVd genome-corresponding sequence); (ii) one or more forward primers for detection of PSTVd of 16 to 30 nucleotides in length, which are designed on the genome-corresponding sequence to locate the 3' end within the region ranging from nucleotide positions 127 to 147 in the PSTVd genome-corresponding sequence; and (iii) one or more forward primers for detection of TCDVd of 16 to 30 nucleotides in length, which are designed on the genome-corresponding sequence to locate the 3' end within the region ranging from nucleotide positions 210 to 224 of the PSTVd genome-corresponding sequence; and determining the presence or absence of nucleic acid amplification by the reverse primer and the forward primer for detection of PSTVd and of nucleic acid amplification by the reverse primer and the forward primer for detection of TCDVd to detect viroid PSTVd and TCDVd in the test plant distinguishing between them.

## Description

### Technical Field

The present invention relates to a method for detecting viroids and a primer set for detecting viroids.

### Background art

Tomato chlorotic dwarf viroid (TCDVd) and potato spindle tuber viroid (PSTVd) infect various host plants, and mainly Solanaceous plants (e.g., potato, tomato, and petunia), causing important plant diseases that produce disease symptoms such as dwarf symptoms in potato plants and yellow or leaf curl symptoms in tomato plants. No incidence of TCDVd had been recently confirmed in Japan; however, infection of tomato plants with TCDVd was confirmed in Hiroshima Prefecture in 2007. Meanwhile, PSTVd is a viroid the incidence of which has not yet been confirmed in Japan, and it is recognized in Japan that vigilance against an invasion of this pathogen is required in view of plant protection.

Viroids are low-molecular-weight nucleic acid pathogens having properties similar to those of viruses and are known to parasitize plants and cause symptoms such as dwarfing and malformation in the plants. A viroid is a single-stranded circular RNA with a molecular weight of about 30,000-810,000 and lacks structural proteins. A viroids is about only one tenth to one hundredth the size of a virus, so that it is difficult to visually confirm the existence of such a viroid using an electron microscope. Moreover, viroids lack structural proteins and thus have no antigenicity. Hence, viroids are detected with difficulty when using general methods for detecting microorganisms or viruses. Accordingly, whether or not a plant is infected with a viroid is often diagnosed by bioassay, by which diagnosis is made based on disease symptoms. The method is problematic in that it may take a long time for disease symptoms to appear, and disease symptoms may differ depending on environmental conditions or cultivars. In recent years, genetic diagnostic methods based on molecular biological detection techniques are increasingly used for detection of viroids (e.g., JP Patent Publication (Kokai) No. 2000-184893 A).

Both viroid PSTVd and TCDVd infect Solanaceous plants such as tomato plants. Hence, techniques for separately detecting the viroids are required. However, PSTVd and TCDVd are related species belonging to the same genus *Pospiviroid* and have 85% or more homology (sequence identity) with each other in terms of full genomic sequence. They are indistinguishable using most conventional methods. Meanwhile, an RT-PCR assay for detecting the genus *Pospiviroid*, to which PSTVd and TCDVd belong, and an RT-PCR assay for separately detecting PSTVd and TCDVd are known (Rudra P. Singh, et al., Journal of General Virology (1999), 80, 2823-2828). However, these methods are problematic in that PSTVd and TCDVd are indistinguishable when they are positively detected in the same reaction solution. A multiplex PCR method is known as a technique for amplifying a plurality of target nucleic acids in a single reaction mixture. However, multiplex PCR is generally applied with great difficulty when distinguishing between related species, since nonspecific binding of primers tends to occur for nucleic acids of related species having high homology.

### Summary of the Invention

### Problem to Be Solved by the Invention

An object of the present invention is to provide a method for simultaneously detecting PSTVd and TCDVd while distinguishing between the two.

### Means for Solving the Problem

As a result of intensive studies to achieve the above object, the present inventors have prepared a primer set that can achieve amplification of nucleic acid amplification fragments with which PSTVd and TCDVd in the same reaction solution can be easily distinguished from each other. Thus, the present inventors have completed the present invention.

The present invention encompasses the following [1] to [3].
[1] A method for detecting viroids, comprising:
   carrying out nucleic acid amplification reaction using RNA from a test plant sample as a template and a primer set comprising (i) a reverse primer of a sequence of 16 to 30 nucleotides in length within the sequence complement of the nucleotide sequence of nucleotide positions 18 to 114 of SEQ ID NO: 1, (ii) one or more forward primers for detection of PSTVd of 16 to 30 nucleotides in length, which are designed on the nucleotide sequence of SEQ ID NO: 1 to locate the 3' end within the region ranging from nucleotide positions 127 to 147 of SEQ ID NO: 1, and (iii) one or more forward primers for detection of TCDVd of 16 to 30 nucleotides in length, which are designed on the nucleotide sequence of SEQ ID NO: 1 to locate the 3' end within the region ranging from nucleotide positions 210 to 224 of SEQ ID NO: 1; and
   determining the presence or absence of nucleic acid amplification by the reverse primer and the forward primer for detection of PSTVd and of nucleic acid amplification by the reverse primer and the forward primer for detection of TCDVd, to detect viroid PSTVd and TCDVd in the test plant distinguishing between them.
      In a preferred embodiment of the primer set to be used in this method, the reverse primer is an oligonucleotide primer of the nucleotide sequence of SEQ NO: 3, the one or more forward primer for detection of PSTVd comprise at least one primer selected from the group consisting of an oligonucleotide primer of the nucleotide sequence of SEQ ID NO: 15, an oligonucleotide primer of the nucleotide sequence of SEQ ID NO: 16, and an oligonucleotide primer of the nucleotide sequence of SEQ ID NO: 17, and the one or more forward primers for detection of TCDVd comprise an oligonucleotide primer of the nucleotide sequence of SEQ ID NO: 5.
      In another preferred embodiment of the primer set to be used in this method, the reverse primer is an oligonucleotide primer of the nucleotide sequence of SEQ ID NO: 3, the one or more forward primers for detection of PSTVd comprise an oligonucleotide primer of the nucleotide sequence of SEQ ID NO: 4, and the one or more forward primers for detection of TCDVd comprise an oligonucleotide primer of the nucleotide sequence of SEQ ID NO: 5.
      In the method for detecting viroids according to the present invention, the primer set is preferably used in a single reaction solution.
      In the detection method according to the present invention, the test plant is preferably a Solanaceous plant.
      In the method according to the present invention, nucleic acid amplification reaction is preferably RT-PCR comprising a step of reverse transcription and a step of multiplex PCR. In this case, it is preferred that, in the step of reverse transcription, the reverse primer is used for nucleic acid amplification reaction; and, in the step of multiplex PCR, the reverse primer, the one or more forward primers for detection of PSTVd, and the one or more forward primers for detection of TCDVd are used for nucleic acid amplification reaction.
      In the primer set to be used in the method according to the present invention, preferably, at least one of the reverse primer, the forward primers for detection of PSTVd, and the forward primers for detection of TCDVd is a labeled primer.
   [2] A primer set for detection of viroids, PSTVd and TCDVd, comprising a reverse primer of a sequence of 16 to 30 nucleotides in length within the sequence complement of the nucleotide sequence of nucleotide positions 18 to 114 of SEQ ID NO: 1, one or more forward primers for detection of PSTVd of 16 to 30 nucleotides in length, which are designed on the nucleotides sequence of SEQ ID NO: 1 to locate the 3' end within the region ranging from nucleotide positions 127 to 147 of SEQ ID NO: 1, and one or more forward primers for detection of TCDVd of 16 to 30 nucleotides in length, which are designed on the nucleotide sequence of SEQ ID NO: 1 to locate the 3' end within the region ranging from nucleotide positions 210 to 224 of SEQ ID NO: 1.
      In one preferred embodiment of the primer set according to the present invention, the reverse primer is an oligonucleotide primer of the nucleotide sequence of SEQ ID NO: 3, the one or more forward primers for detection of PSTVd comprise at least one primer selected from the group consisting of an oligonucleotide primer of the nucleotide sequence of SEQ ID NO: 15, an oligonucleotide primer of the nucleotide sequence of SEQ ID NO: 16, and an oligonucleotide primer of the nucleotide sequence of SEQ ID NO: 17, and the one or more forward primers for detection of TCDVd comprise an oligonucleotide primer of the nucleotide sequence of SEQ ID NO: 5.
      In another preferred embodiment of the primer set according to the present invention, the reverse primer is an oligonucleotide primer of the nucleotide sequence of SEQ ID NO: 3, the one or more forward primers for detection of PSTVd comprise an oligonucleotide primer of the nucleotide sequence of SEQ ID NO: 4, and the one or more forward primers for detection of TCDVd comprise an oligonucleotide primer of the nucleotide sequence of SEQ ID NO: 5.
      In the primer set according to the present invention, preferably, at least one of the reverse primer, the forward primers for detection of PSTVd, and the forward primers for detection of TCDVd is a labeled primer.
   [3] A kit for detecting viroids, comprising the primer set according to [2] above.
      The present invention further encompasses the following [4] to [6].
   [4] A method for detecting viroids, comprising carrying out nucleic acid amplification reaction using RNA from a test plant sample as a template and a primer set comprising a first oligonucleotide primer of the nucleotide sequence of SEQ ID NO: 3, a second oligonucleotide primer of the nucleotide sequence of SEQ ID NO: 4, and a third oligonucleotide primer of the nucleotide sequence of SEQ ID NO: 5, and determining the presence or absence of nucleic acid amplification by the first oligonucleotide primer and the second oligonucleotide primer and of nucleic acid amplification by the first oligonucleotide primer and the third oligonucleotide primer, to detect viroid PSTVd and TCDVd in the test plant distinguishing them.
      In this method, the primer set is preferably used in a single reaction. The method is particularly preferred for a case in which a test plant is a Solanaceous plant. An example of nucleic acid amplification reaction in the method is preferably RT-PCR comprising a step of reverse transcription and a step of multiplex PCR. Preferably, at least one of the first, second, and third oligonucleotide primers to be used in this method is a labeled primer.
   [5] A primer set for detecting viroids, PSTVd and TCDVd, comprising a first oligonucleotide primer of the nucleotide sequence of SEQ ID NO: 3, a second oligonucleotide primer of the nucleotide sequence of SEQ ID NO: 4, and a third oligonucleotide primer of the nucleotide sequence of SEQ ID NO: 5.
      In the primer set, preferably, at least one of the first, second, and third oligonucleotide primers is a labeled primer.
   [6] A kit for detecting viroids, comprising the primer set of [5] above.

This description includes the disclosure of the description and drawings of Japanese Patent Application No. 2009-133144, from which the present application claims priority.

### Effects of the Invention

According to the method of the present invention, viroids, PSTVd and TCDVd, can be distinguished from each other and simultaneously detected.

### Brief Description of the Drawings

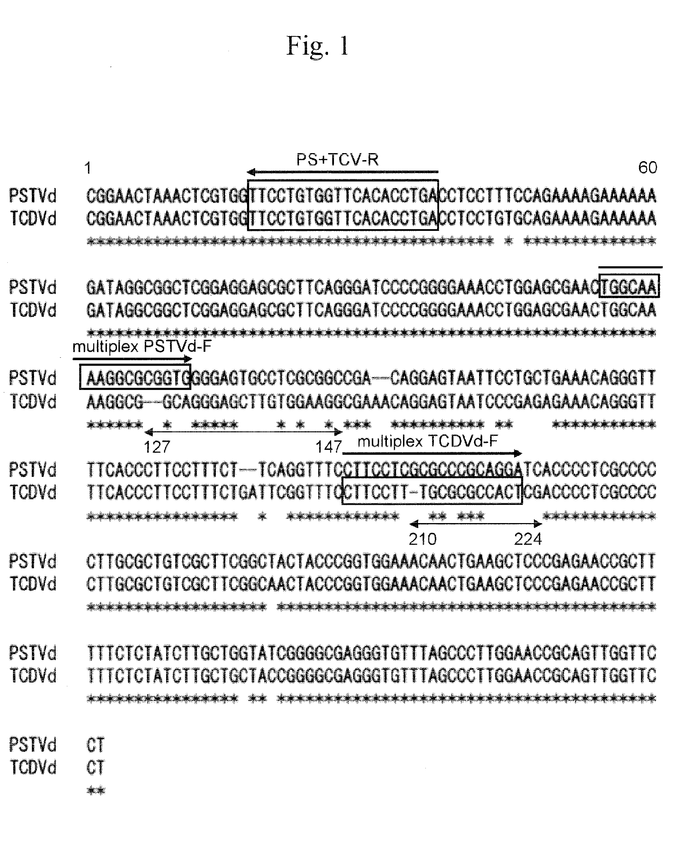

Fig. 1 shows design positions of primers TCDVd-F, PSTVd-F, and PS+TCV-R, on the genomic sequences of TCDVd and PSTVd.

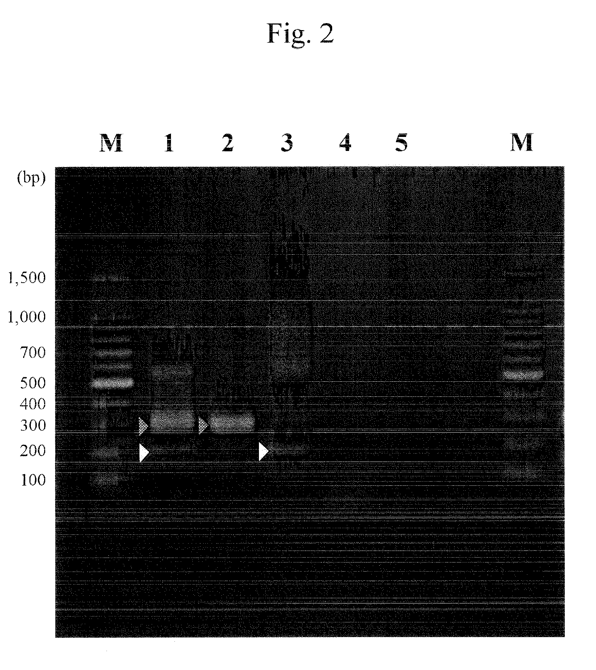

Fig. 2 is an electrophoretic photograph of amplification products resulting from multiplex RT-PCR using the primer set of the primers TCDVd-F, PSTVd-F, and PS+TCV-R. "M" denotes a 100-bp ladder marker (molecular weight marker). For lane 1, TCDVd+PSTVd-mixed RNA was used. For lane 2, PSTVd-RNA was used. For lane 3, TCDVd-RNA was used. For lane 4, an RNA extract from a tomato plant not inoculated with viroids was used. For lane 5, water was used as substitute for a template. Open triangles denote amplification products corresponding to 191 bp and shaded triangles denote amplification products corresponding to 281 bp.

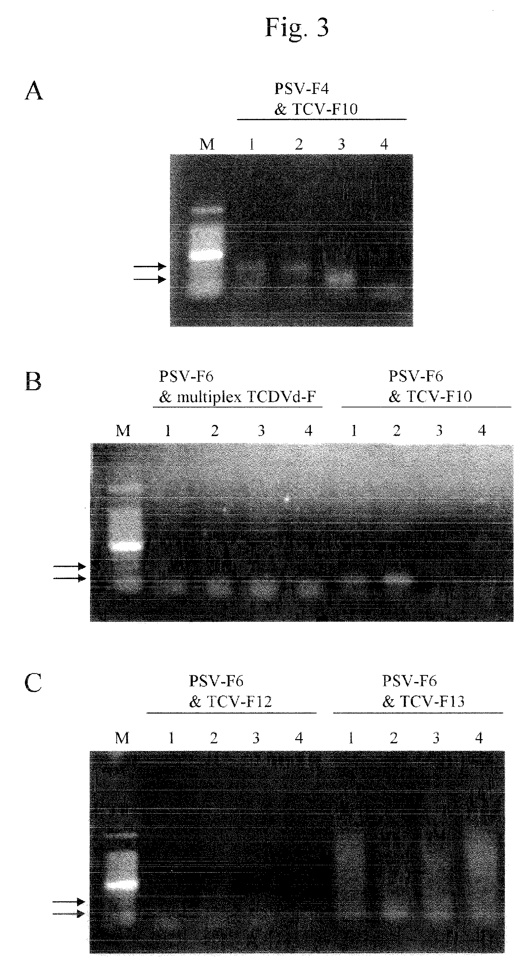

Fig. 3 shows electrophoretic photographs of RT-PCR amplification products amplified from TCDVd and/or PSTVd using combinations of candidate forward primers other than the combination of primers TCDVd-F and PSTVd-F, and a reverse primer PS+TCV-R. Fig. 3A shows the results of amplification using a combination of PSV-F4 and TCV-F10 as forward primers. Fig. 3B shows the results of amplification using as forward primers a combination of PSV-F6 and multiplex TCDVd-F (left panel) and a combination of PSV-F6 and TCV-F10 (right panel). Fig. 3C shows the results of amplification using as forward primers a combination of PSV-F6 and TCV-F12 (left panel) and a combination of PSV-F6 and TCV-F13 (right panel). An upper arrow shown on the left of each panel indicates a 281-bp band specific to PSTVd and a lower arrow indicates the position of a 191-bp band specific to TCDVd. Template RNAs used for each experiment are lane 1, TCDVd+PSTVd-mixed RNA; lane 2, PSTVd-RNA; lane 3, TCDVd-RNA; and lane 4, RNA extract from a tomato plant not inoculated with viroids (healthy individual plant). In Fig. 3, "M" denotes a 100-bp ladder marker (molecular weight marker) as in Fig. 2.

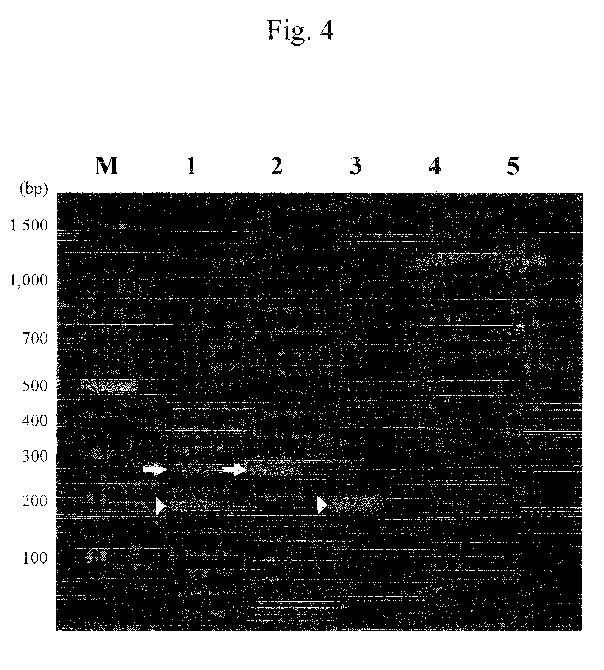

Fig. 4 shows an electrophoretic photograph of amplification products resulting from multiplex RT-PCR using a primer set of a MpR primer (PS+TCV-R), a MpTF primer (TCDVd-F), and forward primers for multiplex detection of PSTVd (MpPTAF, MpPTCF, and MpPCTF). "M" denotes a 100-bp ladder marker (molecular weight marker). Templates used for each experiment are lane 1, TCDVd+PSTVd mixed RNA; lane 2, PSTVd-RNA; lane 3, TCDVd-RNA; lane 4, RNA extract from a healthy tomato plant not inoculated with viroids; and lane 5, RNase-free water. Open triangles denote an amplification product corresponding to 191 bp. White arrows indicate an amplification product corresponding to 270 bp.

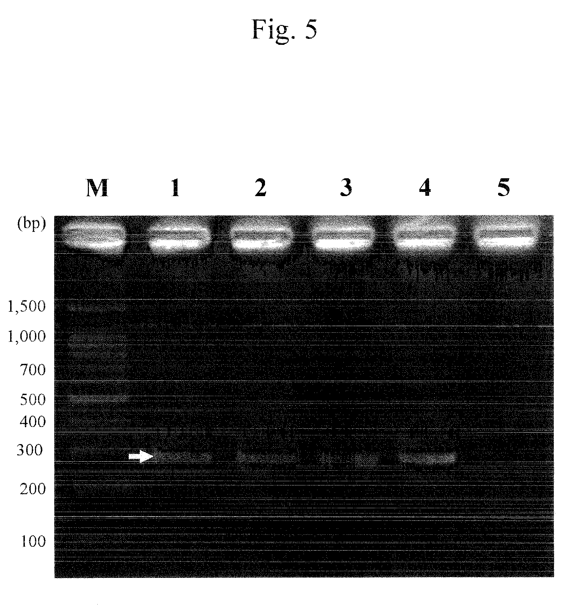

Fig. 5 is a photograph showing the results of an experiment for detection of four patterns of PSTVd variant RNA (PSTVd-UA, PSTVd-UC, PSTVd-CA, and PSTVd-CU) by multiplex RT-PCR using a primer set of a MpR primer (PS+TCV-R), a MpTF primer (TCDVd-F), and forward primers for multiplex detection of PSTVd (MpPTAF, MpPTCF, and MpPCTF). "M", a 100-bp ladder marker (molecular weight marker); lane 1, PSTVd-UA; lane 2, PSTVd-UC; lane 3, PSTVd-CA; lane 4, PSTVd-CU; and lane 5, RNase-free water. White arrow indicates an amplification product corresponding to 270 bp.

### Embodiments for Carrying Out the Invention

Hereinafter, the present invention will be described in detail.

The present invention relates to a method that makes it possible to detect PSTVd and TCDVd while distinguishing between them by: carrying out nucleic acid amplification using a primer set comprising a combination of specific primers described later and test RNA as a template; and then determining the presence or the absence of the amplification of a predetermined amplification product. The full genomic sequence information of PSTVd and TCDVd is available under the Accession Nos. EU862231 and AB329668, respectively, from GenBank sequence database. The full genomic sequence of PSTVd as disclosed under Accession No. EU862231 (denoted by DNA sequence) is shown in SEQ ID NO: 1 and the full genomic sequence of TCDVd as disclosed under Accession No. AB329668 (denoted by a DNA sequence) is shown in SEQ ID NO: 2.

In the methods of the present invention, any test RNA can be used as a template. Preferably, RNA that contains or may contain RNA) pathogens, potato spindle tuber viroid (PSTVd) and/or tomato chlorotic dwarf viroid (TCDVd) is used as a template. In the method of the present invention, RNA from a test plant sample to be tested for the presence of or infection with PSTVd or TCDVd can be used as a template when carrying the test.

Test plants to be subjected to the method of the present invention are not limited, but are preferably plants that are sensitive to PSTVd and TCDVd infection. Examples of such test plants include plants of the family Solanaceous (Solanaceous plants) (e.g., plants of the genus *Solanum*, the genus *Petunia*, the genus *Capsicum*, or the genus *Tobacco*), plants of the family Verbenaceae (e.g., plants of the genus *Verbena*), plants of the family Asteraceae (e.g., plants of the genus *Chrysanthemum* or the genus *Glebionis*). Specific examples of the test plants include potato, tomato, petunia, bell pepper, tobacco, verbena, and crown daisy. Plants that may be infected with PSTVd or TCDVd are particularly suitable subjects to which the method of the present invention is applicable.

A test plant sample may be a whole test plant body or a part of the plant body removed therefrom (e.g., leaves, stems, fruits, calyces, petals, roots, tubers, or seeds), and may also be cells from them or a cell culture thereof (e.g., cultured cells and callus).

An example of RNA from a test plant sample is not limited, but is, DNA extract from a test plant sample (e.g., total RNA) or a purified preparation from the extract, for example. RNA can be extracted from a test plant sample according to an RNA extraction technique as generally employed in the art of plant molecular biology. For example, any known RNA extraction method can be used, such as a single-step RNA purification method, a glass adsorption method, and an acid phenol extraction method. RNA can also be extracted using a commercially available RNA extraction reagent such as TRIzol^{(R)} reagent (Invitrogen), RNAiso (TaKaRa), RNeasy^{™} (QIAGEN), or ToTALLY RNA^{™} (Ambion) or a commercially available RNA extraction kit (e.g., TRIzol^{(R)} Plus RNA purification kit (Invitrogen), or Maxwell^{™} 16 Total RNA Purification Kit (Promega)). The thus extracted RNA may be purified by a well-known technique such as HPLC purification or the like if necessary, before the RNA is subjected to RT-PCR.

In a preferred embodiment of the present invention, nucleic acid amplification is carried out using a predetermined primer set and RNA from a test plant sample as a template. Nucleic acid amplification can be carried out according to any nucleic acid amplification method, using RNA as a template (e.g., RT-PCR, a NASBA method, and a Ribo-SPIA^{™} amplification method). Particularly, reverse transcription PCR (RT-PCR) is preferably carried out. RT-PCR generally comprises a step of reverse transcription for synthesizing cDNA from template RNA using reverse transcriptase and first strand cDNA synthesis primers, and a PCR (polymerase chain reaction) step for amplifying the cDNA (first strand cDNA) synthesized in the step of reverse transcription. Regarding detailed procedures for RT-PCR, textbooks in the art of molecular biology, such as Sambrook, J. and RUSSEL, D. W., (2001), Molecular Cloning: A Laboratory Manual, Cold Sparing Harbor Laboratory Press, can be referred to. In the method of the present invention, as nucleic acid amplification, RT-PCR comprising a reverse transcription step and a multiplex PCR step is particularly appropriately carried out. The term "multiplex PCR" in the context of the present invention refers to PCR for which a primer set comprising 2 or more types of forward primers is used in a single reaction solution. The term "primer set" in the context of the present invention refers to a combination of 1, 2, or more types of forward primers and reverse primers. Even if the primer set according to the present invention comprises one type of reverse primer, the primer set can be used for multiplex PCR, as long as separate amplification products are produced with combinations (as primer pairs) of the reverse primer and each of 2 or more types of forward primers in the primer set.

In the method of the present invention, nucleic acid amplification is carried out, so that an amplification product of interest may be directly amplified using a predetermined primer set from test plant sample-derived RNA or an amplification product of interest may be amplified using a predetermined primer set from the DNA (cDNA) that has been reverse transcribes from the RNA.

The primer set to be used in the method of the present invention is specifically a primer set for multiplex PCR comprising the following primers (i) to (iii):
(i) a reverse primer of a sequence of 16 to 30 nucleotides in length within the sequence complement of the nucleotide sequence of nucleotide positions 18 to 114 of SEQ ID NO: 1;
(ii) one or more forward primers for detection of PSTVd of 16 to 30 nucleotides in length, which are designed on the nucleotide sequence of SEQ ID NO: 1 to locate the 3' end within the region ranging from nucleotide positions 127 to 147 of SEQ ID NO: 1; and
(iii) one or more forward primers for detection of TCDVd of 16 to 30 nucleotides in length, which are designed on the nucleotide sequence of SEQ ID NO: 1 to locate the 3' end within, the region ranging from nucleotide positions 210 to 224 of SEQ ID NO: 1.

Here, the term, "..primer(s), which are/is designed on the nucleotide sequence of SEQ ID NO: 1" means an oligonucleotide primer that comprises a part of nucleotides sequence of SEQ ID NO: 1 and has a sequence designed so as to hybridize under stringent conditions to a polynucleotide consisting of the sequence complementary to the nucleotide sequence of SEQ ID NO: 1. The term "stringent conditions" refers to, for example, conditions under which washing is carried out in 1 x SSC and 0.1% SDS at 60°C. A primer that is designed on the nucleotide sequence of SEQ ID NO: 1 may consist of a sequence of continuous 16 to 30 nucleotides from the nucleotide sequence of SEQ ID NO: 1 or may be of a nucleotide sequence having 80% or more, preferably 85% or more, further preferably 90% or more sequence identity with a sequence of continuous 16 to 30 nucleotides from the nucleotide sequence of SEQ ID NO: 1.

When the primer set is used, detection of a specific amplification product produced by the reverse primer and the forward primer for detection of PSTVd indicates the presence of PSTVd. Furthermore, detection of a specific amplification product produced by the reverse primer and the forward primer for detection of TCDVd indicates the presence of TCDVd.

In addition, in the present invention, when one type of reverse primer, one type of forward primer for detection of PSTVd, and one type of forward primer for detection of TCDVd are used, they may be referred to as "first primer," "second primer," and "third primer," respectively.

In a preferred embodiment of the primer set to be used in the method of the present invention, the reverse primer of (i) above is preferably an oligonucleotide primer of the nucleotide sequence of SEQ ID NO: 3. The primer set according to the present invention preferably comprises one type of the reverse primer of (i) above, but the example is not limited to the above primer set.

In the primer set according to the present invention, one or more forward primers for detection of PSTVd preferably comprise at least one, preferably 2 or more, and more preferably all primers selected from the group consisting of an oligonucleotide primer of the nucleotide sequence of SEQ ID NO: 15, an oligonucleotide primer of the nucleotide sequence of SEQ ID NO: 16, and an oligonucleotide primer of the nucleotide sequence of SEQ ID NO: 17.

Moreover, in the primer set according to the present invention, one or more forward primers for detection of TCDVd preferably comprise an oligonucleotide primer of the nucleotide sequence of SEQ ID NO: 5.

In a further preferred embodiment, the primer set can be used for detection of viroids, wherein, as the reverse primer of (i), an oligonucleotide primer of the nucleotide sequence of SEQ ID NO: 3 is used; as one or more forward primers of (ii) for detection of PSTVd, an oligonucleotide primer of the nucleotide sequence of SEQ ID NO: 15, an oligonucleotide primer of the nucleotide sequence of SEQ ID NO: 16, and an oligonucleotide primer of the nucleotides sequence of SEQ ID NO: 17 are used; and as one or more forward primers of (iii) for detection of TCDVd, an oligonucleotide primer of the nucleotides sequence of SEQ ID NO: 5 is used. With the use of the primer set, various PSTVd lines having any one of 4 patterns ("UA," "UC," "CA," and "CU'") as a genomic sequence corresponding to nucleotide positions 140-141 of SEQ ID NO: 1 can be broadly detected.

In another preferred embodiment, the primer set to be used in the method of the present invention is a primer set comprising: an oligonucleotide primer of the nucleotide sequence of SEQ ID NO: 3 as the reverse primer of (i) (first primer: 5'-TCAGGTGTGAACCACAGGAA-3'); an oligonucleotide primer of the nucleotide sequence of SEQ ID NO: 4 as one or more forward primers of (ii) for detection of PSTVd (second primer: 5'-TGGCAAAAGGCGCGGTG-3'); and an oligonucleotide primer of the nucleotide sequence of SEQ ID NO: 5 as one or more forward primers of (iii) for detection of TCDVd (third primer: 5'-CTTCCTTTGCGCGCCACT-3').

In the context of the present invention, oligonucleotide primers are conveniently defined by referring to the DNA sequence shown in any of SEQ ID NOS: 1, 3-5, and 15-17, but "oligonucleotide primer" include not only DNA primers, but also RNA primers. When the oligonucleotide primer is RNA, "T (thymine)" in the reference DNA sequence is read as "U (uracil)." Examples of the oligonucleotide primer of the present invention also include a chimera of DNA and RNA. The oligonucleotide primer of the present invention may contain natural nucleotides alone, but may also contain modified nucleotides. Examples of such modified nucleotides include, but are not limited to, deoxyinosine, deoxyuracil, and phosphorothioated nucleotide. Oligonucleotide primers of the present invention containing modified nucleotides are encompassed within the range of the oligonucleotide primer as defined by the nucleotide sequence denoted by natural nucleotides corresponding to the modified nucleotides. Such oligonucleotide primer can be synthesized by persons skilled in the art according to a conventional method such as a phosphoroamidite method. For example, such oligonucleotide primer can also be chemically synthesized using a commercially available automated oligonucleotide synthesizer.

In the present invention, at least one of the reverse primer, the forward primer(s) for detection of PSTVd, and the forward primer(s) for detection of TCDVd contained in the above primer set is preferably a labeled primer that is obtained by addition of a labeling substance to the relevant oligonucleotide. When a primer set comprising one type of reverse primer (first primer), one type of forward primer for detection of PSTVd (second primer), and one type of forward primer for detection of TCDVd (third primer) is used, in particular, for example, when a primer set comprising the first oligonucleotide primer of the nucleotide sequence of SEQ 10 NO: 3, the second oligonucleotide primer of the nucleotide sequence of SEQ ID NO: 4, and the third oligonucleotide primer of the nucleotide sequence of SEQ ID NO: 5 is used, at least one of the first, second, and third oligonucleotide primers in the primer set is preferably a labeled primer that is obtained by addition of a labeling substance to the oligonucleotide. A labeling substance is generally added to the 5' end or 3' end of an oligonucleotide. As labeling substances, various labeling substances that are generally used in the art of molecular biology, biochemistry, and the like can be used. Examples of such labeling substances include fluorescent molecules, dye molecules, radioisotopes, biotin, digoxigenin, a phosphate group, an amino group, peptide moieties of peptide nucleic acid (PNA), and tag sequences. The use of a labeled primer can further facilitate detection, purification, and the like of the resulting amplification products.

In the method of the present invention, when nucleic acid amplification is carried out by RT-PCR, for example, the reverse transcription step can be carried out according to a conventional method using reverse first strand cDNA synthesis primers, and the like. Viroids are circular RNAs. Hence, for reverse transcription, a reverse primer of a sequence complement which is specific to PSTVd and TCDVd is preferably used as a first, strand synthesis primer. As such first strand can synthesis primer, a reverse primer of a sequence of 16 to 30 nucleotides and more preferably 18 to 25 nucleotides within the sequence complement of the nucleotide sequence of nucleotide positions 18 to 114 of SEQ ID NOS: 1 and 2 which show the full genomic sequences of PSTVd and TCDVd are preferred. In the primer set according to the present invention, such reverse primer can also be used for nucleic acid amplification in combination with a forward primer for detection of PSTVd and a forward primer for detection of TCDVd. A first strand cDNA synthesis primer, which is particularly preferable in view of convenience, sensitivity, and the like, is an oligonucleotide primer of the nucleotide sequence of SEQ ID NO: 3 that may be contained in the above primer set. In the present invention, the term "sequence complement" means a sequence consisting of a nucleotide sequence complementary to the full-length of a given nucleotide sequence. Reverse transcription reaction can be carried out by treating a reverse transcription reaction solution containing template RNA, first strand DNA synthesis primers, reverse transcriptase, dNTPs, and the like at 42°C for 30 minutes and then 99°C for 5 minutes, and then keeping the resultant at 4°C. However, the procedures are not limited to them.

When RT-PCR is carried out, the above primer set is preferably used in the PCR step following the reverse transcription step. In this case, a PCR, reaction mixture containing the above primer set comprising the reverse primer, theforward primer(s) for detection of PSTVd, and the forward primer(s) for detection of TCDVd (or the above primer set comprising the first to third primers), a template cDNA obtained in the reverse transcription step, DNA polymerise, dNTPs, and the like is prepared. The reaction mixture is then treated for a plurality of cycles at a temperature for nucleic acid denaturation, an annealing temperature, and an extension temperature, so that PCR can be carried out. Specific examples of appropriate reaction conditions are described in the Examples below, for example, cycling conditions under which the reaction mixture is subjected to 3 minutes of treatment at 98°C; 35 cycles of 98°C for 45 seconds, 62°C for 10 seconds, and 74°C for 45 seconds, followed by 74°C for 5 minutes, and then the resultant is preferably maintained at 4°C. When the reverse primer of the primer set (e.g., an oligonucleotide primer of the nucleotide sequence of SEQ ID NO: 3 was used as a first strand DNA synthesis primer in the reverse transcription step, another addition of the first primer is not required upon preparation of a PCR reaction mixture. This is because if the PCR reaction mixture is prepared using an unpurified reaction solution (an unpurified reverse transcription product) after completion of reverse transcription reaction, the reverse primer generally remains in the PCR reaction mixture. For RT-PCR that is carried out as described above, the reverse primer is used for nucleic acid amplification in the reverse transcription step, and the reverse primer same as that used in the reverse transcription step, one or more forward primers for detection of PSTVd and one or more forward primers for detection of TCDVd are used for nucleic acid amplification in the subsequent multiplex PCR step.

In the present invention, after nucleic acid amplification is carried out using the above primer set, whether or not PSTVd-specific nucleic acid amplification occurs (i.e., the presence or absence of nucleic acid amplification by the reverse primer and the forward primer for detection of PSTVd in the primer set) and whether or not TCDVd-specific nucleic acid amplification occurs (i.e., the presence or absence of nucleic acid amplification by the reverse primer and the forward primer for detection of TCDVd in the primer set) are determined, thereby detecting PSTVd and TCDVd.

The presence or absence of such nucleic acid amplifications can be determined by, but not limited to, examining whether or not a nucleic acid fragment specifically amplified by the reverse primer and the forward primer for detection of PSTVd; and a nucleic acid fragment specifically amplified using the reverse primer and the forward primer for detection of TCDVd are produced. For example, if an amplification product, which is obtained with the reverse primer and any one of the one or more forward primers for detection of PSTVd, is positively detected in the reaction mixture, nucleic acid amplification by these primers is determined to be "present." This result indicates that PSTVd has been detected in the test plant. On the other hand, if an amplification product, which is obtained using the reverse primer and any one of the one or more forward primers for detection of TCDVd, is positively detected in the reaction mixture, nucleic acid amplification by these primers is determined to be "present." This result indicates that the TCDVd has been detected in the test plant. In contrast, if neither of those amplification products is detected in the reaction mixture, nucleic acid amplifications by those primers are determined to be "absent." The result indicates that neither of the two viroids has been detected in the test plant.

An amplification fragment generated by the reverse primer and the forward primer for detection of PSTVd and an amplification fragment generated by the reverse primer and the forward primer for detection of TCDVd can be detected using any method for detection of nucleic acids. Example of such method for detection of nucleic acids include, but are not limited to, gel electrophoresis analysis, capillary electrophoresis analysis, sequencing analysis using an autosequencer or the like, and MALD1-TOF/MS analysis.

The size of an amplification fragment obtained using the reverse primer and the forward primer for detection of PSTVd or the forward primer for detection of TCDVd can be predicted by persons skilled in the art on the basis of the nucleotide sequences of SEQ ID NO: 1 (PSTVd genome) and SEQ ID NO: 2 (TCDVd gnome). A viroid is a circular RNA, and thus a region consisting of a sequence ranging from nucleotide position 1 of SEQ ID NO: 1 (and 2) to the nucleotide position at which the 5' end of a reverse primer is designed and a sequence ranging from the nucleotide position at which the 5' terminus of the forward primer for detection of PSTVd or of the forward primer for detection of TCDVd is designed on the sequence of SEQ ID NO: 1 (and 2), to nucleotide position 358 (for PSTVd) or 359 (for TCDVd) of SEQ ID NO: 1 (and 2) is amplified as a single continuous sequence.

For example, when a primer set of: an oligonucleotide primer of the nucleotide sequence of SEQ ID NO: 3 as a reverse primer; at least one primer (and preferably the three primers) selected from the group consisting of an oligonucleotide primer of the nucleotide sequence of SEQ ID NO: 15, an oligonucleotide primer of the nucleotide sequence of SEQ ID NO: 16, and an oligonucleotide primer the nucleotide sequence of SEQ ID NO: 17, as one or more forward primers for detection of PSTVd; and an oligonucleotide primer of the nucleotide sequence of SEQ ID NO: 5 as one or more forward primers for detection of TCDVd, is used, a 270-bp fragment is amplified from PSTVd RNA, while 191-bp fragment is amplified from TCDVd RNA.

However, an amplification, fragment by the reverse primer and the forward primer for detection of PSTVd and an amplification fragment by the reverse primer and the forward primer for detection of TCDVd can be somewhat shorter or longer (by 1 to 5 nucleotides, for example, but the number of nucleotides is not limited thereto) than the above expected size if a mutation such as deletion or addition is present in viroid RNA (PSTVd RNA or TCDVd RNA) to be detected, for example. Even if such a mutation is present, for example, if a clear amplification band is detected around the above expected size in electrophoresis analysis, it can be determined that an amplification product of interest can be positively detected. Alternatively or in addition to, sequencing is carried out for the thus obtained amplification fragment and then the nucleotide sequence is compared with the nucleotide sequence shown in SEQ ID NO: 1, so that it can be clearly and easily determined whether or not the resulting amplification fragment is a fragment generated by the reverse primer and the forward primer for detection of PSTVd or a fragment generated by the reverse primer and the forward primer for detection of TCDVd.

In a preferred embodiment, a reaction mixture after completion of the reaction of nucleic acid amplification is subjected to electrophoresis analysis, such as 1% agarose gel electrophoresis analysis in 1 x TBE buffer, and then the size of the amplification fragment is compared with those of molecular weight marker, to determine the presence or absence of nucleic acid amplification. As a result, when a clear amplification band corresponding to the expected size (e.g., 270 bp in the above example) of an amplification fragment generated by the reverse primer and the forward primer for detection of PSTVd is detected, it is determined that nucleic acid amplification using the reverse primer and the forward primer for detection, of PSTVd has occurred. In that case, it can be concluded that PSTVd has been detected. On the other hand, if a clear amplification band corresponding to the expected size (e.g., 191 bp in the above example) of an amplification fragment generated by the reverse primer and the forward primer for detection of TCDVd is detected, it is determined that nucleic acid amplification using the reverse primer and the forward primer for detection of TCDVd has occurred. In that case, it can be concluded that TCDVd has been detected. If both an amplification band of a size specific to PSTVD and an amplification band of a size specific to TCDVd are detected, it can be concluded that both PSTVd and TCDVd have been successfully detected. These amplification bands can be easily observed as clearly different amplification band patterns with the use of electrophoresis gel (e.g., 1% agarose gel) by which relatively short amplification fragments can be sufficiently separated.

Moreover in one embodiment of the present invention, when one type of reverse primer, one type of forward primer for detection of PSTVd, and one type of forward primer for detection of TCDVd are used, and specifically, for example, when the first oligonucleotide primer of the nucleotide sequence of SEQ ID NO: 3, the second oligonucleotide primer of the nucleotide sequence of SEQ ID NO: 4, and the third oligonucleotide primer of the nucleotide sequence of SEQ ID NO: 5 are used, PSTVd and TCDVd are detected by carrying out nucleic acid amplification using the above primer set and then determining whether or not PSTVd-specific nucleic acid amplification has occurred (i.e., the presence or absence of nucleic acid amplification by the first oligonucleotide primer and the second oligonucleotide primer) and TCDVd-specific nucleic acid amplification has occurred (i.e., the presence or the absence of nucleic acid amplification by the first oligonucleotide primer and the third oligonucleotide primer).

The presence or absence of such nucleic acid amplifications can be determined by, but not limited to, examining whether or not a specific amplification product has been generated using the first primer (SEQ ID NO: 3) and the second primer (SEQ ID NO: 4) and whether or not a specific amplification product has been generated using the first primer (SEQ ID NO: 3) and the third primer (SEQ ID NO: 5). For example, if a specific amplification product generated by the first primer and the second primer is positively detected in the reaction mixture, nucleic acid amplification by these primers is determined to be "present," and this result indicates that PSTVd has been detected in the test plant. On the other hand, if a specific amplification product generated by the first primer and the third primer is positively detected in the reaction mixture, nucleic acid amplification by these primers is determined to be "positive," and the result indicates that TCDVd has been detected in the test plant. In contrast, if neither of those specific amplification products is detected in the reaction mixture, nucleic acid amplifications by those primers are determined to be "absent." The result indicates that neither of the two viroids has been detected in the test plant.

A specific amplification product generated by the first primer and the second primer, and a specific amplification product generated by the first primer and the third primer can be detected using any method for detection of DNA. Examples of such DNA detection method include, but are not limited to, gel electrophoresis analysis, capillary electrophoresis analysis, sequencing analysis using an auto sequencer or the like, and MALDI-TOF/MS analysis.

A specific amplification product generated by the first primer and the second primer is typically a 281-bp nucleic acid amplification fragment of the nucleotide sequence of a region of nucleotide positions 115 to 358 and nucleotide positions 1 to 37 of SEQ ID NO: 1 (PSTVd sequence) (actually the region is continuous on circular viroid RNA).

However, the nucleotide sequence of the amplification fragment can contain some mutations in the nucleotide sequence when a detection subject is a further variant of PSTVd. Accordingly, when a nucleotide deletion or addition is present as a mutation, the amplification fragment can be somewhat shorter or longer than 281 bp (e.g., by 1 to 5 nucleotides, but the example is not limited thereto). Even it such a mutation is present, if a clear amplification bans is detected around 281 bp by electrophoresis analysis, for example, it can be determined that a specific amplification product generated by the first primer and the second primer has been positively detected. Alternatively, the nucleotide sequence of the thus obtained amplification fragment is determined and then compared with the nucleotide sequence shown in SEQ ID NO: 1, so that it can be clearly and easily determined whether or not the amplification fragment is a specific amplification product by the first primer and the second primer from PSTVd.

Meanwhile, a specific amplification product generated by the first primer and the third primer is typically a 191-bp nucleic acid amplification fragment of the nucleotide sequence of a region of nucleotide positions 206 to 359 and nucleotide positions 1 to 37 of SEQ ID NO: 2 (TCDVd sequence) (actually the region is continuous on circular viroid RNA). However, the nucleotide sequence of the amplification fragment can contain some mutations in the nucleotide sequence when a detection subject is a TCDVd variant, for example. Accordingly, when a nucleotide deletion or addition is present as a mutation, the amplification fragment can be somewhat shorter or longer than 191 bp (e.g., by 1 to 5 nucleotides, but the number of nucleotides is not limited thereto). Even if such a mutation is present, if a clear amplification band is detected at around 191 bp by electrophoresis analysis, for example, it can be determined that a specific amplification product generated by the first primer and the third primer has been positively detected. Alternatively, the nucleotide sequence of the thus obtained amplification fragment is determined and then compared with the nucleotide sequence shown in SEQ ID NO: 2, so that it can be clearly and easily determined whether or not the amplification fragment is a specific amplification product the first primer and the third primer from TCDVd.

In a preferred embodiment, a reaction mixture after completion of the reaction of nucleic acid amplification is subjected to electrophoresis analysis, such as 1% agarose gel electrophoresis analysis in 1 x TBE buffer, and then the size of the amplification fragment is compared with those of molecular weight marker determine the presence or absence of nucleic acid amplification. As a result, when a clear amplification band corresponding to 281 bp is detected, it is determined that nucleic acid amplification using the first primer and the second primer has occurred. In that case, it can be concluded that PSTVd has been detected. On the other hand, if a clear amplification band corresponding to 191 bp is detected, it is determined that nucleic acid amplification using the first primer and the third primer has occurred. In that case, it can be concluded that TCDVd has been detected. If both a clear amplification band corresponding to 281 bp and an amplification band corresponding to 191 bp are detected, it can be concluded that both PSTVD and TCDVd have been successfully detected. These amplification bands corresponding to 281 bp and 191 bp, respectively, can be easily observed as clearly different amplification band patterns with the use of electrophoresis gel (e.g., 1% agarose gel) by which relatively short amplification fragments can be sufficiently separated.

In the present invention, as described above, PSTVd and TCDVd in a test plant can be detected while distinguishing between them based on the result of determination of the presence or absence of nucleic acid amplifications by the above 2 or more primer pairs contained in a primer set. With the use of the detection method of the present invention, PSTVd and TCDVd are distinguishable from each other.

The method for detecting viroids of the present invention is particularly suitable for, but not limited to, a case where nucleic acid amplification is carried out using the above primer set in a single reaction. In such nucleic acid amplification, nucleic acid amplification reaction (e.g., PCR) can be separately carried out in a reaction mixture containing the reverse primer and the forward primer for detection of PSTVd, and another reaction mixture containing the reverse primer and the forward primer for detection of TCDVd. However, it is more preferable to carry out multiplex PCR using a single reaction mixture containing the reverse primer, the forward primer for detection of PSTVd, and the forward primer detection of TCDVd which make up a primer set. In the present invention, when the first oligonucleotide primer of the nucleotide sequence of SEQ ID NO: 3, the second oligonucleotide primer of the nucleotide sequence of SEQ ID NO: 4, and the third oligonucleotide primer of the nucleotide sequence of SEQ ID NO: 5, for example, are used in nucleic acid amplification, separate nucleic acid amplification reactions (e.g., PCR) can be carried out using a primer pair of the first primer and the second primer in a reaction mixture and a primer pair of the first primer and the third primer in another reaction solution, but more preferably, PCR is carried out in a single reaction using one reaction mixture containing the first, the second, and the third primers.

In general, multiplex PCR, for which 3 or more types of primers are used simultaneously in a single reaction, is convenient, since it enables simultaneous analysis. However, specific reaction conditions differ depending on each primer, and therefore multiplex PCR is often associated with difficulty in obtaining a plurality of specific amplification products. In particular, when a plurality of primers specific to each of closely related species are used for multiplex PCR, possibility of cross-reaction, increases. Hence, it becomes further difficult to obtain specific amplification products. However, in the method of the present invention, even if primers specific to PSTVd and TCDVd, respectively, which are closely related species having high sequence homology from each other, are simultaneously used in a single reaction, both PSTVd and TCDVd can be specifically amplified and each viroid can be distinguished from each other with high sensitivity and detected. Furthermore, according to the method of the present invention, PSTVd and TCDVd each can be specifically detected with high sensitivity even for a sample containing, both PSTVd and TCDVd as template RNAs.

The present invention also provides the above-mentioned primer set that is used in the detection method of the present invention. The primer set according to the present invention is a primer set for detecting viroid PSTVd and TCDVd comprising (i) a reverse primer of a sequence of 16 to 30 nucleotide in length within a sequence complement of the nucleotide sequence of nucleotide positions 18 to 114 of SEQ ID NO: 1, (ii) one or more forward primers for detection of PSTVd of 16 to 30 nucleotides in length, which are designed on the nucleotide sequence of SEQ ID NO: 1 to locate the 3' end within a region ranging from nucleotide positions 127 to 147 of SEQ ID NO: 1, (iii) one or more forward primers for detection of TCDVd of 16 to 30 nucleotides in length, which are designed on the nucleotide sequence of SEQ ID NO: 1 to locate the 3' end within a region ranging from nucleotide positions 210 to 224 of SEQ ID NO: 1.

The primer set according to the present invention may be a primer set comprising: a reverse primer that is an oligonucleotide primer of the nucleotide sequence of SEQ ID NO: 3; one or more forward primers for detection of PSTVd that comprise at least one primer (or preferably all primers) selected from the group consisting of an oligonucleotide primer of the nucleotide sequence of SEQ ID NO: 15, an oligonucleotide primer of the nucleotide sequence of SEQ ID NO: 16, and an oligonucleotide primer of the nucleotide sequence of SEQ ID NO: 17; and one or more forward primers for detection of TCDVd that comprise an oligonucleotide primer of the nucleotide sequence of SEQ ID NO: 5, for example. The primer set enables detection of various PSTVd lines while distinguishing from TCDVd. Each primer contained in the primer set according to the present invention is as specifically described above. For example, each primer may be a labeled primer. A primer set, in which at least one of the reverse primer, the forward primer(s) for detection of PSTVd, and the forward primer(s) for detection of TCDVd is a labeled primer, may also be a preferable example of the primer set of the present invention. The primer set according to the present invention is particularly suitable for detection of viroid PSTVd and TCDVd based on nucleic acid amplification, for example, RT-PCR comprising the step of reverse transcription and the step of multiplex PCR.

The primer set according to the present invention may also be a primer set comprising an oligonucleotide primer (first primer) of the nucleotide sequence of SEQ ID NO: 3, an oligonucleotide primer (second primer) of the nucleotide sequence of SEQ ID NO: 4, and an oligonucleotide primer (third primer) of the nucleotide sequence of SEQ ID NO: 5, for example. The primer set is particularly suitable for, but not limited to, detection of PSTVd and TCDVd. Oligonucleotide primers contained in the primer set are as described above and may be labeled primers, for example. A primer set comprising the first primer, the second primer, and the third primer above, at least one of which is a labeled primer, is a preferred example of the primer set of the present invention. The primer set according to the present invention is particularly suitable for detection of PSTVd and TCDVd based on nucleic acid amplification, for example, RT-PCR comprising the step of reverse transcription and the step of multiplex PCR.

The present invention also provides a kit for detecting viroids, which comprises the above-mentioned primer set according to the present invention. The kit according to the present invention may be a kit for detecting PSTVd and TCDVd or may be a kit with which various viroids including PSTVd and TCDVd can be detected. The kit for detecting viroids of the present invention may further comprise other reagents, containers, instructions, and the like. The kit may comprise reverse transcription reagents and/or and reagents four nucleic acid amplification such as reverse transcriptase, DNA polymerase, a dNTP mixture, and a nucleic acid amplification reaction buffer, a reagent for RNA extraction and the like, for example. The kit for detecting viroids of the present invention may also comprise reagents for detecting viroids, such as other oligonucleotide primers specific to various viroids (e.g., viroids infective for Solanaceous plants), for example.

### Examples

The present invention is further illustrated with reference to the following examples. However, these examples do not limit the technical scope of the present invention.

### [Example 1]

### 1. RNA extraction

RNA were extracted from tomato leaves infected with a potato spindle tuber viroid (PSTVd) alone and tomato leaves infected with a tomato chlorotic dwarf viroid (TCDVd) alone by the following procedures.

First, 1 mL of TRIzol^{(R)} reagent (Invitrogen) was added to 50 mg to 100 mg of the above-mentioned infected leaves and then the resultant was ground. Chloroform (200 mL) was added to the resultant, and then the mixture was vortexed and then subjected to 15 minutes of centrifugation at 12000 g. The thus obtained supernatant was collected and then 200 mL of isopropanol was added to and mixed with the supernatant, followed by 10 minutes of centrifugation at 12000 g. The supernatant was discarded, 1 mL of 75% ethanol was added, and then centrifugation was carried out for 5 minutes at 7500 g. All centrifugation steps during the above procedures were carried out at 2°C to 8°C. The thus obtained precipitate was dried and then 50 mL to 100 mL of RNA free water (Sigma) was added depending on concentration. The solution containing total RNA was used as a template for the reverse transcription reaction (RT-PCR) described later.

### 2. Primer design and RT-PCR

Candidate multiplex RT-PCR primers for detecting viroid PSTVd and TCDVd distinguishing between PSTVd and TCDVd were designed, using a computer, on the full genomic sequences of PSTVd and TCDVd (shown in SEQ ID NOS: 1 and 2, respectively). The present inventors focused on specific regions (a region ranging from nucleotide positions 127 to 147 and a region ranging from 210 to 224 of SEQ ID NO: 1 which shows the full genomic sequence of PSTVd) from among low-homology regions of both viroids. They designed a candidate primer for detection of PSTVd and a candidate primer for detection of TCDVd to be used as forward primers such that the 3' ends were located within the regions. A candidate primer for detection of PSTVd (5'-TGGCAAAAGGCGCGGTG-3' (SEQ ID NO: 4)) designed on the nucleotide sequence ranging from nucleotide positions 115 to 131 of the full genomic sequence of PSTVd (SEQ ID NO: 1) was designated was the multiplex primer PSTVd-F (see, Fig. 1). Also, a candidate primer (5'-CTTCCTTTGCGCGCCACT-3' (SEQ ID NO: 5)) for detection of TCDVd designed on the nucleotide sequence ranging from nucleotide positions 206 to 223 of the genome sequence of TCDVd (SEQ ID NO: 2) was designated as multiplex primer TCDVd-F (see, Fig. 1). As such designed primers, DNA primers synthesized by a conventional method were used.

Furthermore, a reverse primer (5'-TCAGGTGTAACCACAGGAA-3' (SEQ ID NO: 3)) designed on a sequence complement of the region of nucleotide positions 18 to 37 of the full genomic sequences (SEQ ID NOS: 1 and 2) of PSTVd and TCDVd was designated as multiplex reverse primer PS+TCV-R. The reverse primer synthesized as a DNA primer by a. conventional method was used as a first strand cDNA synthesis primer for reverse transcription reaction (RT) of RT-PCR. Furthermore, the reverse primer remaining in the reaction solution after reverse transcription reaction was used as a PCR primer for the subsequent PCR step.

A reverse transcription reaction mixture was prepared with the following composition.

| Composition of reverse transcription reaction mixture | |
|---|---|
| RT buffer (TOYOBO) | 2 µl |
| dNTPs (10 mM) (TOYOBO) | 1 µl |
| RNase inhibitor (1 U) (TOYOBO) | 0.5 µl |
| Reverse transcriptase RverTra Ace^{(R)} (20 µM) (TOYOBO) | 0.5 µl |
| Multiplex reverse primer PS+TCV-R (20 µM) | 0.5 µl |
| RNase free water | 4.5 µl |
| Total RNA (template) | 1 µl |
| Total | 10 µl |

As total RNA, 3 types of template were used herein- The 3 types of template are: specifically, total RNA from leaves infected with TCDVd (TCDVd-RNA) alone; total RNA from leaves infected with PSTVd (PSTVd-RNA) alone; and a mixture of the two RNAs prepared by mixing them in equivalent amounts (TCDVd+PSTVd mixed RNA).

Reverse transcription reaction was carried out by treating the above reaction mixture to 42°C four 30 minutes and then to 99°C for 5 minutes, and then storing the mixture at 4°C. After completion of the reverse transcription reaction, 1 µl of the reverse transcription product was put into a PCR tube and thus a PCR reaction mixture with the following composition was prepared.

| Composition of PCR reaction mixture | |
|---|---|
| KOD Dash buffer (TOYOBO) | 1 µl |
| polymerase KOD Dash (TOYOBO) | 0.1 µl |
| dNTPs (2 mM) (TOYOBO) | 1 µl |
| Multiplex primer PSTVd-F (10 µM) | 0.1 µl |
| Multiplex primer TCDVd-F (10 µM) | 0.1 µl |
| Water | 6.7 µl |
| Reverse transcription (RT) product (with template cDNA and primer PS+TCV-R) | 1 µl |
| Total | 10 µl |

PCR was carried out with denaturation treatment for 3 minutes at 98°C, and 35 cycles of 45 seconds at 98°C, 10 seconds at 62°C and 45 seconds at 74°C, followed by treatment for 5 minutes at 74°C and then storage at 4°C.

After completion of PCR, one drop of the PCR product was mixed with one drop of a loading dye. The mixture was loaded onto 1% agarose gel and then electrophoresis was carried out in 1 x TBE buffer. After electrophoresis, the dye was caused to emit light using UV light, and the presence or the absence of an amplification band was determined.

Fig. 2 shows the results. Multiplex RT-PCR was carried out as described above using both viroid RNAs. As a result, a clear specific band corresponding to the expected size of 191 bp (lane 3) was observed for TCDVd-RNA and a clear specific band corresponding to the expected size of 281 bp (lane 2) was observed for PSTVd-RNA. The 191-bp band for TCDVd-RNA was an amplification fragment corresponding to the region ranging from nucleotide positions 206 to 359 and nucleotide positions 1 to 37 (actually the region was consecutive on circular viroid RNA) of SEQ ID NO: 2 (see, open triangles in Fig. 2). Similarly, the 281-bp band for PSTVd-RNA was an amplification fragment corresponding to the region ranging from nucleotide positions 115 to 358 and nucleotide positions 1 to 37 (actually the region was consecutive on circular viroid RNA) of SEQ ID NO: 1 (see, shaded triangles in Fig. 2).

As shown in Fig. 2, both bands (191 bp and 281 bp) specific to TCDVd and PSTVd, respectively, were observed for TCDVd+PSTVd mixed RNA (lane 1), the band (28 bp) specific to PSTVd was observed for PSTVD-RNA (lane 2), and the band (191 bp) specific to TCDVd was observed for TCDVd-RNA (lane 3). On the other hand, none of these bands were observed for RNA from healthy tomato leaves not inoculated with any viroid (lane 4) and water alone (lane 5), as controls.

As described above, it was demonstrated that the presence of TCDVd and PSTVd could be clearly distinguished from each other by RT-PCR using the above the multiplex primer TCDVd-F, multiplex primer PSTVd-F, and multiplex reverse primer PS+TCV-R. In this method, a band specific to either TCDVd or PSTVd alone was amplified, but almost no band common to both viroids was amplified. It was thus demonstrated that the method rarely shows false positive results.

Moreover, detection was carried out in a similar manner as that used above by varying the annealing temperatures (55°C, 58°C, 60°C, and 62°C) and the final concentrations of the forward primers (0.2 µM, 0.1 µM, and 0.05 µM) for PCR in RT-PCR. Thus, RT-PCR conditions more appropriate for simultaneous detection of both viroids were examined. As a result, it was demonstrated that, in view of detection sensitivity, the most appropriate annealing temperature for PCR was 62°C: and the most appropriate final concentration of each forward primer for PCR was 0.1 µM.

### [Example 2]

For comparison with the detection results in Example 1, PSTVd and TCDVd were detected by carrying out multiplex RT-PCR by procedures and conditions similar to or Example 1 using other several types of candidate primers for detection of PSTVd and several types of candidate primers for detection of TCDVd, which had been designed at positions analogous to those of multiplex primers, PSTVd-F and TCDVd-F.

For reverse transcription reaction, the same multiplex reverse primer PS+TCV-R as that used in Example 1 was used. For PCR, candidate primers for detection of PSTVd and candidate primers for detection of TCDVd (shown in Table 1) were used in various combinations (Table 2) in addition to the multiplex reverse primer PS+TCV-R. In Table 1 and Table 2, "multiplex PSTVd-F" denotes "multiplex primer PSTVd-F" and "multiplex TCDVd-F" denotes "multiplex primer TCDVd-F."

**Table 1 Candidate Multiplex RT-PCR primers**

| Use | | Primer name | Sequence (5'-3') | Nucleotide position * |
|---|---|---|---|---|
| Reverse primer | | PS+TCV-R | TCAGGTGTGAACCACAGGAA (SEQ ID NO: 3) | 18-37 |
| Forward primer | Candidate Primer for detection of PSTVd | Multiplex PSTVD-F | TGGCAAAAGGCGCGGTG (SEQ ID NO: 4) | 115-131 |
| | | PSV-F2 | CTGGCAAAAGGCGCGGTG (SEQ ID NO:6) | 114-131 |
| | | PSV -F3 | ACTGGCAAAAGGCGCGGTG (SEQ ID NO:7) | 113-131 |
| | | PSV-F4 | TGGCAAAAGGCGCGGTGG (SEQ ID NO:8) | 115-132 |
| | | PSV-F5 | AACTGGCAAAAGGCGCGGTG (SEQ ID NO: 9) | 112-131 |
| | | PSV-F6 | CTGGCAAAAGGCGCGGTGG (SEQ ID NO: 10) | 114-132 |
| | Candidate Primer for detection of TCDVd | Multiplex TCDVd-F | CTTCCTTTGCGCGCCACT (SEQ ID NO: 5) | 206-223 |
| | | TCV-F10 | TTCCTTTGCGCGCCACTT (SEQ ID NO: 11) | 207-223 |
| | | TCV-F11 | CTTCCTTTGCGCGCCACTC (SEQ ID NO:12) | 206-224 |
| | | TCV-F12 | CCTTCCTTTGCGCGCCACT (SEQ ID NO:13) | 205-223 |
| | | TCV-F13 | CTTCCTTTGCGCGCCACTCC (SEQ ID NO: 14) | 206-225 |

| | | | | |
|---|---|---|---|---|
| *Nucleotide positions of the candidate primers for detection of PSTVd are based on the nucleotide sequence shown in SEQ ID NO: 1. Nucleotide positions of the candidate primers for detection of TCDVd are based on the nucleotide sequence shown in SEQ ID NO:2. Nucleotide position of the reverse primer PS+TCV-R is based on both the nucleotide sequences of SEQ ID NOS: 1 and 2. | | | | |

**Table 2 Combinations of tested PCR forward primers and detection results**

| | | Candidate Primer for detection of TCDVD | | | | |
|---|---|---|---|---|---|---|
| | | Multiplex TCDVd-F | TCV-F10 | TCV-F11 | TCV-F12 | TCV-F13 |
| Candudate Primer for detection of PSTVD | Multiplex PSTVD-F | P&T | E | E | E | E |
| | PSV-F2 | E | E | E | E | E |
| | PSV-F3 | E | (nt) | (nt) | (nt) | (nt) |
| | PSV-F4 | E | E | E | E | E |
| | PSV-F5 | E | (nt) | (nt) | (nt) | (nt) |
| | PSV-F6 | E | E | E | E | E |

| | | | | | | |
|---|---|---|---|---|---|---|
| P&T: Bands specific to TCDVd and PSTVd, respectively, were detected. E: Only a band specific to either TCDVd or PSTVd was detected or neither of bands specific to TCDVd or PSTVd, respectively, was clearly detected. (nt): Not tested | | | | | | |

Table 2 shows amplification results obtained by the above RT-PCR. As shown in Table 2, in the case of the combination of multiplex PSTVd-F and multiplex TCDVd-F, amplification bands specific to TCDVd and PSTVd, respectively, could be amplified in one reaction. It was thus demonstrated that TCDVd and PSTVd can be positively detected distinguishing from each other (see, P&T in Table 2). On the other hand, in the case of combinations of candidate primers other than these primers, no clear specific band was amplified or a band specific to either TCDVd or PSTVD alone could be amplified, and both TCDVd and PSTVd could not be positively detected. In particular, when a mixed RNA of TCDVD and PSTVd was used as a template, amplification bands specific to TCDVd and PSTVd, respectively, were clearly detected using the combination of multiplex PSTVd-F and multiplex TCDVd-F, while in contrast, in the case of the combinations of candidate forward primers other than these primers, only amplification bands much unclearer than that in the case of detection using either TCDVd or PSTVd RNA alone as a template were detected.

As an example, electrophoretic photographs showing some of the results shown in Table 2 are shown in Fig. 3. In the case of RT-PCR using the combinations of candidate forward primers shown in Fig. 3, clear specific amplification bands could not be detected for either TCDVd RNA) or PSTVd RNA or both templates. Furthermore, when a mixed RNA of TCDVd and PSTVd was used as a template, neither of amplification bands specific to them was clearly detected (Fig. 3). It was demonstrated by the results that the combinations of the above candidate primers other than the combination of multiplex PSTVd-F and multiplex TCDVd-F are: unsuitable for the method for detecting TCDVd and PSTVd distinguishing between them; and unsuitable for such detection of TCDVd and PSTVd in an RNA sample suspected of containing both TCDVd and PSTVd in particular.

Therefore, it was revealed that for a test for detection of TCDVd and PSTVd based on multiplex RT-PCR, a combination of multiplex PSTVd-F and multiplex TCDVd-F is an optimal combination of forward primers. The full-length nucleotide sequences of TCDVd and PSTVd have 85% or more homology with each other. Hence, it was predicted that preparation of a multiplex primer set that enables positive detection of viroid TCDVd and PSTV distinguishing them would be very difficult. It was actually demonstrated that among many combinations of candidate primers designed at analogous positions, only a primer set comprising the above-mentioned combination of multiplex PSTVd-F and multiplex TCDVd-F is an optimal primer set for multiplex RT-PCR by which both TCDVD and PSTVd can each be specifically and positively detected.

The above-used primer set (multiplex primer PSTVd-F, multiplex primer TCDVd-F, and reverse primer PS+TCV-R) according to the present invention was designed such that the primers contained matched nucleotides in many various variants reported for PSTVd and TCDVd, and thus the primer set can be broadly used for distinguishing between PSTVd and TCDVd. Specifically, each primer of the primer set according to the present invention was designed in view of, in addition to the sequence of SEQ ID NO: 1 of PSTVd and the sequence of SEQ ID NO: 2 of TCDVd, the nucleotide sequences of previously reported TCDVd variants (e.g., GenBank Accession No. DQ859013 [Plant Dis., 91, p.324 (2007)], AF162131 [J. Gen. Virol., 80, p.2823-2828 (1999)], EF582392 [Plant Pathol., 57, p.400 (2008)], EF582393 [Plant Pathol., 57, p.400 (2008)], and AY372399 [Eur. J. Plant Pathol., 110, p.823-831 (2004)]) and the nucleotide sequences of previously reported PSTVd variants (e.g., GenBank Accession No. Z34272 [EMBO J., 13 (24), p. 6172-6177 (1994)], M25199 [Nucleic Acids Res., 10 (24), p.7947-7957 (1982)], AF458986 [J. Gen. Virol., 84, p. 751-756 (2003)], AF459005 [Virology, 187 (2), p.654-662 (1992)], A937179 [J. Gen. Virol., 86, p.1835-1839 (2005)], M88677 [EMBO J., 4, p. 2181-2190 (1985)], M88678 [Nature, 273 (5659), p. 203-208 (1978)], M88681 [EMBO J., 4, p. 2181-2190 (1985)], U23058 [Nature, 273 (5659), p.203-208 (1978)], U23059 [Nature, 273(5659), p. 203-208 (1978)], V01465 [Nature, 273 (5659), p. 203-208 (1978)], X97387 [Virology, 226(2), p. 191-197 (1996)], and M16826 [Proc. Natl. Acad. Sci. U.S.A., 84, p. 3967-3971 (1987)]). Hence, the primer set can be used for distinguishing among many variants including these variants.

Based on the above results, it was demonstrated that PSTVd and TCDVd can be clearly distinguished and detected by carrying out RT-PCR using the multiplex primer TCDVd-F, the multiplex primer PSTVd-F, and the multiplex reverse primer PS+TCV-R, and confirming the presence or absence of a TCDVd-specific amplification fragment (corresponding to the 191-bp band) and a PSTVd-specific amplification fragment (corresponding to the 281-bp band).

### [Example 3]

PSTVd has many lines. To make it possible to detect even a larger number of PSTVd lines, preparation of a primer set for simultaneous detection of PSTVd and TCDVd was further attempted by further designing a sequence of a primer for detection of PSTVd, which is to be used as a forward primer, such that the 3 end is located within the region ranging from nucleotide positions 127 to 147 of SEQ ID NO: 1 showing the full genomic sequence of PSTVd as in Examples 1. Multiplex RT-PCR was carried out in a manner similar to that in Example 1 using new candidate primers for detection of PSTVd, and it was examined if simultaneous detection of PSTVd and TCDVd was possible.

Examples of the good detection results for PSTVd and TCDVd using specific primer sets comprising the thus newly designed forward primer for detection of PSTVd are as described below.

First, as a template for reverse transcription reaction, each RNA of PSTVd and TCDVd, which had been extracted by the same method as that in Example 1 was used. Reverse transcription reaction was carried out by the same procedures as and under the same conditions as those employed in Example 1 using the same multiplex reverse primer (hereinafter, also referred to as MpR) PS+TCV-R (SEQ ID NO: 3) as that in Example 1.

For the subsequent PCR, a combination of the multiplex reverse primer PS+TCV-R, the forward primer for detection of PSTVd, and the forward primer for detection of TCDVd was used as a multiplex PCR primer set (Table 3). As a multiplex forward primer for detection of TCDVd (hereinafter, may also be referred to MpTF), the same multiplex primer TCDVd-F (SEQ ID NO: 5) as in that of Example 1 was used. To broadly detect various PSTVd lines, 3 types of multiplex forward primer for detection of PSTVd having different 3' terminal sequences were newly designed and used. These 3' terminal sequences of the multiplex forward primers for detection of PSTVd were 3 patterns, "A," "TC," and "CT," so that the sequence ranging from nucleotide positions 140 to 141 of SEQ ID NO: 1, differing among various lines of PSTVd, could be detected.

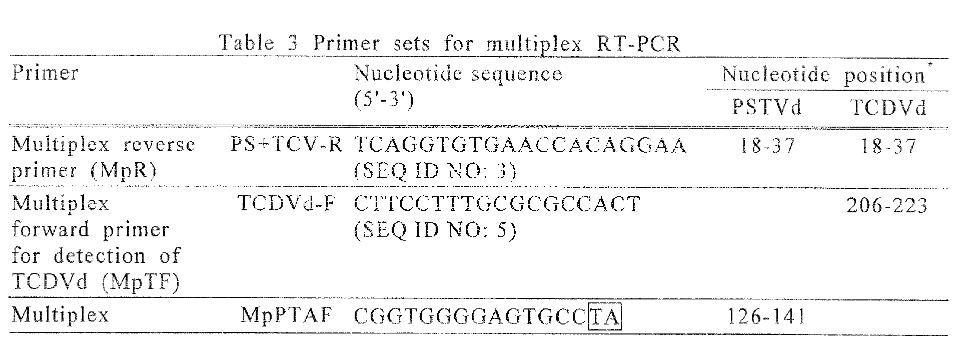

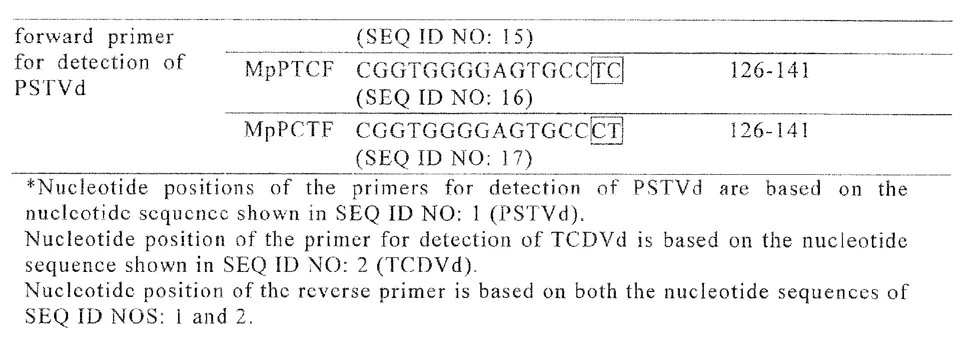

A PCR. reaction mixture with the following composition was prepared.

| Composition of PCR reaction mixture | |
|---|---|
| KOD Dash buffer (TOYOBO) | 1 µl |
| DNA polymerase KOD Dash (TOYOBO) | 0.1 µl |
| dNTPs (2 mM) (TOYOBO) | 1 µl |
| MpTF primer (TCDVd-F) (10 µM) | 0.1 µl |
| Primer MpPTAF (10 µM) | 0.1 µl |
| Primer MpPTCF (10 µM) | 0.1 µl |
| Primer MpPCTF (10 µM) | 0.1 µl |
| Water | 6.5 µl |
| Reverse transcription (RT) product (with template cDNA and primer PS+TCV-R) | 1 µl |
| Total | 10 µl |

PCR was carried out in a manner similar to that in Example 1, with denaturation treatment for 3 minutes at 98°C, and 35 cycles of 45 seconds at 98°C, 10 seconds at 62°C, and 45 seconds at 74°C, followed by treatment for 5 minutes at 74°C and then storage at 4°C.

After completion of PCR, one drop each of the PCR product was mixed with one drop each of a leading dye. The mixture was loaded onto 1% agarose gel and then electrophoresis was carried out in 1 x TBE buffer. After electrophoresis, the dye was caused to emit light using UV light, and the presence or the absence of an amplification band was determined.

Fig. 4 shows the results. Multiplex RT-PCR was carried out as described above using both viroid RNAs, and as a result, a clear specific band corresponding to the expected size of 191 bp (lane 3) was observed for TCDVd-RNA and a clear specific band corresponding to the expected size of 270 bp (lane 2) was observed for PSTVd-RNA. The 191-bp band for TCDVd-RNA was an amplification fragment corresponding to the region ranging from nucleotide positions 206 to 359 and nucleotide positions 1 to 37 (actually the region was consecutive on circular viroid RNA) of SEQ ID NO: 2 (see, open triangles in Fig. 4). Similarly, the 270-bp band for PSTVd-RNA was an amplification fragment corresponding to the region ranging from nucleotide positions 126 to 358 and nucleotide positions 1 to 37 (actually the region was continuous on circular viroid RNA) of SEQ ID NO: 1 (see, white arrows in Fig. 4).

As shown in Fig. 4, both bands (191 bp and 270 bp) specific to TCDVd and PSTVd, respectively, could be observed for the TCDVd+PSTVd mixed RNA sample (lane 1), the band (270 bp) specific to PSTVd could be observed for the PSTVd-RNA sample (lane 2), and the band (191 bp) specific to TCDVd could be observed for the TCDVd-RNA sample (lane 3). On the other hand, none of these bands were observed for RNA from healthy tomato leaves not inoculated with any viroid (lane 4) and water alone (lane 5), as controls.

Further testing was conducted to confirm whether various PSTVd lines could be detected using multiplex RT-PCR primer set shown in Table 3. In the sequence ranging from nucleotide positions 140 to 141 of SEQ ID NO: 1 in PSTVd, 4 patterns ("UA," "UC," "CA," and "CU") exist, depending on PSTVd lines. 4 patterns of PSTVd variant RNAs, in which the genomic sequence corresponding to the nucleotide positions 140-141 of SEQ ID NO: 1 is "UA," "UC," "CA," or "CU," were produced (named PSTVd-UA, PSTVd-UC, PSTVd-CA, and PSTVd-CU, respectively) using the PSTVd line, X76844. Detection tests based on multiplex RT-PCR were conducted for the produced RNAs as templates with the same procedures and conditions described above using the multiplex RT-PCR primer set (Table 3) comprising a mixture of MpPTAF, MpTCF, and MpCTF primers. As a result, as shown in Fig. 5, amplification products of the same size (270 bp) were observed for all 4 patterns of PSTVd variant RNAs using the multiplex RT-PCR primer set. That is, all of the 4 patterns of PSTVd variant RNAs could be detected (lanes 1-4). Meanwhile, no band was observed for the control sample using water alone (lane 5).

Thus, it was demonstrated that the presence of TCDVd and the presence of PSTVd can be clearly distinguished by multiplex RT-PCR using the primer set composed of the multiplex reverse primer PS+TCV-R, the multiplex forward primer (TCDVd-F) for detection of TCDVd, and the multiplex forward primers (MpPTAF, MpTCF, and MpCTF) for detection of PSTVd, 1 and various PSTVd lines can be detected. Also, with the use of this method, bands specific to TCDVd or PSTVd, respectively, were amplified, but almost no specific bands common to both viroids were amplified. Hence, it was demonstrated that the multiplex RT-PCR primer set is also optimum for simultaneous detection of TCDVd and PSTVd.

### Industrial Applicability

The method of the present invention can be used for detecting the pathogens, viroid TCDVd and PSTVd, that infect mainly Solanaceous plants, while distinguishing between them. With the use of the methods of the present invention, TCDVd and PSTVd can be distinguished from each other in a single reaction system. The method of the present invention not only contributes to protection against the invasion of the country by TCDVd and PSTVd, but also is applicable to strengthening of plant protection systems via use for rapid diagnosis upon domestic quarantine.

All publications, patents, and patent applications cited herein are incorporated herein by reference in their entirety.

### Sequence Listing Free Text

SEQ ID NOS: 3-17: primers.

## Claims

1. A method for detecting viroids, comprising:
carrying out nucleic acid amplification reaction using RNA from a test plant sample as a template and a primer set comprising (i) a reverse primer of a sequence of 16 to 30 nucleotides in length within the sequence complement of the nucleotide sequence of nucleotide positions 18 to 114 of SEQ ID NO: 1, (ii) one or more forward primers for detection of PSTVd of 16 to 30 nucleotides in length, which are designed on the nucleotide sequence of SEQ ID NO: 1 to locate the 3' end within the region ranging from nucleotide positions 127 to 147 of SEQ ID NO: 1, and (iii) one or more forward primers for detection of TCDVd of 16 to 30 nucleotides in length, which are designed on the nucleotide sequence of SEQ ID NO: 1 to locate the 3' end within the region ranging from nucleotide positions 210 to 224 of SEQ ID NO: 1; and
determining the presence or absence of nucleic acid amplification by the reverse primer and the forward primer for detection of PSTVd and of nucleic acid amplification by the reverse primer and the forward primer for detection of TCDVd, to detect viroid PSTVd and TCDVd in the test plant distinguishing between them.

2. The method according to claim 1, wherein the reverse primer is an oligonucleotide primer of the nucleotide sequence of SEQ ID NO: 3, the one or more forward primers for detection of PSTVd comprise at least one primer selected from the group consisting of an oligonucleotide primer of the nucleotide sequence of SEQ ID NO: 15, an oligonucleotide primer of the nucleotide sequence of SEQ ID NO: 16, and an oligonucleotide primer of the nucleotide sequence of SEQ ID NO: 17, and the one or more forward primers for detection of TCDVd comprise an oligonucleotide primer of the nucleotide sequence of SEQ ID NO: 5.

3. The method according to claim 1, wherein the reverse primer is an oligonucleotide primer of the nucleotide sequence of SEQ ID NO: 3, the one or more forward primers for detection of PSTVd comprise an oligonucleotide primer of the nucleotide sequences of SEQ ID NO: 4, and the one or more forward primers for detection of TCDVd comprise an oligonucleotide primer of the nucleotide sequence of SEQ ID NO: 5.

4. The method according to any one or claims 1 to 3, wherein the primer set is used in a single reaction solution.

5. The method according to any one of claims 1 to 4, wherein the test plant is a Solanaceous plant.

6. The method according to any one of claims 1 to 5, wherein the nucleic acid amplification reaction is RT-PCR comprising a step of reverse transcription and a step of multiplex PCR.

7. The method according to claim 6, wherein, in the step of reverse transcription, the reverse primer is used for nucleic acid amplification reaction; and, in the step of multiplex PCR, the reverse primer, the one or more forward primers for detection of PSTVd, and the one or more forward primers for detection of TCDVd are used for nucleic acid amplification reaction.

8. The method according to any one of claims 1 to 7, wherein at least one of the reverse primer, the forward primers for detection of PSTVd, and the forward primers for detection of TCDVd is a labeled primer.

9. A primer set for detection of viroids, PSTVd and TCDVd, comprising (i) a reverse primer of a sequence of 16 to 30 nucleotides in length within the sequence complement of the nucleotide sequence of nucleotide positions 18 to 114 of SEQ ID NO: 1, (ii) one or more forward primers for detection of PSTVd of 16 to 30 nucleotides in length, which are designed on the nucleotide sequence of SEQ ID NO: 1 to locate the 3' end within the region ranging from nucleotide positions 127 to 147 of SEQ ID NO: 1, and (iii) one or more forward primers for detection of TCDVd of 16 to 30 nucleotides in length, which are designed on the nucleotide sequence of SEQ ID NO: 1 to locate the 3' end within the region ranging from nucleotide positions 210 to 224 of SEQ ID NO: 1.

10. The primer set according to claim 9, wherein the reverse primer is an oligonucleotide primer of the nucleotide sequence of SEQ ID NO: 3, the one or more forward primers for detection of PSTVd comprise at least one primer selected from the group consisting of an oligonucleotide primer of the nucleotide sequence of SEQ ID NO: 15, an oligonucleotide primer of the nucleotide sequence of SEQ ID NO: 16, and an oligonucleotide primer of the nucleotide sequence of SEQ ID NO: 17, and the one or more forward primers for detection of TCDVd comprise an oligonucleotide primer of the nucleotide sequence of SEQ ID NO: 5.

11. The primer set according to claim 9, wherein the reverse primer is an oligonucleotide primer of the nucleotide sequence of SEQ ID NO: 3, the one or more forward primers for detection of PSTVd comprise an oligonucleotide primer of the nucleotide sequence of SEQ ID NO: 4, and the one or more forward primers for detection of TCDVd comprise an oligonucleotide primer of the nucleotide sequence of SEQ ID NO: 5.

12. The primer set according to any one of claims 9 to 11, wherein at least one of the reverse primer, the forward primers for detection of PSTVd, and the forward primers for detection of TCDVd is a labeled primer.

13. A kit for detecting viroids, comprising the primer set according to any one of claims 9 to 12.


SEQUENCE LISTING

<110>  Incorporated Administrative Agency National Agriculture and Food Research
Organization

<120>  Method of simultaneous detection of viroids TCDVd and PSTVd

<130>  PH-4044-PCT

<150>  JP 2009-133144
<151>  2009-06-02

<160>  17

<170>  PatentIn version 3.4


<210>  1
<211>  358
<212>  DNA
<213>  Potato spindle tuber viroid PSTVd

<220>
<223>  Inventor: Matsushita, Yousuke; Tsuda, Shinya

<400>  1
cggaactaaa ctcgtggttc ctgtggttca cacctgacct cctttccaga aaagaaaaaa     60

gataggcggc tcggaggagc gcttcaggga tccccgggga aacctggagc gaactggcaa    120

aaggcgcggt ggggagtgcc tcgcggccga caggagtaat tcctgctgaa acagggtttt    180

cacccttcct ttcttcaggt ttccttcctc gcgcccgcag gatcacccct cgcccccttg    240

cgctgtcgct tcggctacta cccggtggaa acaactgaag ctcccgagaa ccgctttttc    300

tctatcttgc tggtatcggg gcgagggtgt ttagcccttg gaaccgcagt tggttcct      358


<210>  2
<211>  359
<212>  DNA
<213>  Tomato chlorotic dwarf viroid TCDVd

<400>  2
cggaactaaa ctcgtggttc ctgtggttca cacctgacct cctgtgcaga aaagaaaaaa     60

gataggcggc tcggaggagc gcttcaggga tccccgggga aacctggagc gaactggcaa    120

aaggcggcag ggagcttgtg gaaggcgaaa caggagtaat cccgagagaa acagggtttt    180

cacccttcct ttctgattcg gtttccttcc tttgcgcgcc actcgacccc tcgccccctt    240

gcgctgtcgc ttcggcaact acccggtgga aacaactgaa gctcccgaga accgcttttt    300

ctctatcttg ctgctaccgg ggcgagggtg tttagccctt ggaaccgcag ttggttcct     359


<210>  3
<211>  20

<212>  DNA
<213>  Artificial

<220>
<223>  Primer

<400>  3
tcaggtgtga accacaggaa                                                    20


<210>  4
<211>  17
<212>  DNA
<213>  Artificial

<220>
<223>  primer

<400>  4
tggcaaaagg cgcggtg                                                       17


<210>  5
<211>  18
<212>  DNA
<213>  Artificial

<220>
<223>  primer

<400>  5
cttcctttgc gcgccact                                                      18


<210>  6
<211>  18
<212>  DNA
<213>  Artificial

<220>
<223>  primer

<400>  6
ctggcaaaag gcgcggtg                                                      18


<210>  7
<211>  19
<212>  DNA
<213>  Artificial

<220>
<223>  primer

<400>  7
actggcaaaa ggcgcggtg                                                     19


<210>  8
<211>  18
<212>  DNA

<213>  Artificial

<220>
<223>  primer

<400>  8
tggcaaaagg cgcggtgg                                                    18


<210>  9
<211>  20
<212>  DNA
<213>  Artificial

<220>
<223>  primer

<400>  9
aactggcaaa aggcgcggtg                                                  20


<210>  10
<211>  19
<212>  DNA
<213>  Artificial

<220>
<223>  primer

<400>  10
ctggcaaaag gcgcggtgg                                                   19


<210>  11
<211>  17
<212>  DNA
<213>  Artificial

<220>
<223>  primer

<400>  11
ttcctttgcg cgccact                                                     17


<210>  12
<211>  19
<212>  DNA
<213>  Artificial

<220>
<223>  primer

<400>  12
cttcctttgc gcgccactc                                                   19


<210>  13
<211>  19
<212>  DNA
<213>  Artificial

<220>
<223>  primer

<400>  13
ccttcctttg cgcgccact                                                    19


<210>  14
<211>  20
<212>  DNA
<213>  Artificial

<220>
<223>  primer

<400>  14
cttcctttgc gcgccactcg                                                   20


<210>  15
<211>  16
<212>  DNA
<213>  Artificial

<220>
<223>  primer

<400>  15
cggtggggag tgccta                                                       16


<210>  16
<211>  16
<212>  DNA
<213>  Artificial

<220>
<223>  primer

<400>  16
cggtggggag tgcctc                                                       16


<210>  17
<211>  16
<212>  DNA
<213>  Artificial

<220>
<223>  primer

<400>  17
cggtggggag tgccct                                                       16

SEQUENCE LISTING

<110>  Incorporated Administrative Agency National Agriculture and Food
Research Organization

<120>  Method of simultaneous detection of viroids TCDVd and PSTVd

<130>  PH-4044-PCT

<140>  PCT/JP2010/058894
<141>  2010-05-26

<150>  JP 2009-133144
<151>  2009-06-02

<160>  17

<170>  PatentIn version 3.4


<210>  1
<211>  358
<212>  DNA
<213>  Potato spindle tuber viroid PSTVd

<220>
<223>  Inventor: Matsushita, Yousuke; Tsuda, Shinya

<400>  1
cggaactaaa ctcgtggttc ctgtggttca cacctgacct cctttccaga aaagaaaaaa      60

gataggcggc tcggaggagc gcttcaggga tccccgggga aacctggagc gaactggcaa     120

aaggcgcggt ggggagtgcc tcgcggccga caggagtaat tcctgctgaa acagggtttt     180

cacccttcct ttcttcaggt ttccttcctc gcgcccgcag gatcacccct cgcccccttg     240

cgctgtcgct tcggctacta cccggtggaa acaactgaag ctcccgagaa ccgctttttc     300

tctatcttgc tggtatcggg gcgagggtgt ttagcccttg gaaccgcagt tggttcct       358


<210>  2

<211>  359
<212>  DNA
<213>  Tomato chlorotic dwarf viroid TCDVd

<400>  2
cggaactaaa ctcgtggttc ctgtggttca cacctgacct cctgtgcaga aaagaaaaaa      60

gataggcggc tcggaggagc gcttcaggga tccccgggga aacctggagc gaactggcaa     120

aaggcggcag ggagcttgtg gaaggcgaaa caggagtaat cccgagagaa acagggtttt     180

cacccttcct ttctgattcg gtttccttcc tttgcgcgcc actcgacccc tcgccccctt     240

gcgctgtcgc ttcggcaact acccggtgga aacaactgaa gctcccgaga accgcttttt     300

ctctatcttg ctgctaccgg ggcgagggtg tttagccctt ggaaccgcag ttggttcct      359


<210>  3
<211>  20
<212>  DNA
<213>  Artificial

<220>
<223>  Primer

<400>  3
tcaggtgtga accacaggaa                                                  20


<210>  4
<211>  17
<212>  DNA
<213>  Artificial

<220>
<223>  primer

<400>  4
tggcaaaagg cgcggtg                                                     17


<210>  5
<211>  18

<212>  DNA
<213>  Artificial

<220>
<223>  primer

<400>  5
cttcctttgc gcgccact                                                    18


<210>  6
<211>  18
<212>  DNA
<213>  Artificial

<220>
<223>  primer

<400>  6
ctggcaaaag gcgcggtg                                                    18


<210>  7
<211>  19
<212>  DNA
<213>  Artificial

<220>
<223>  primer

<400>  7
actggcaaaa ggcgcggtg                                                   19


<210>  8
<211>  18
<212>  DNA
<213>  Artificial

<220>
<223>  primer

<400>  8
tggcaaaagg cgcggtgg                                                    18

<210> 9
<211> 20
<212> DNA
<213> Artificial

<220>
<223> primer

<400> 9
aactggcaaa aggcgcggtg 20


<210> 10
<211> 19
<212> DNA
<213> Artificial

<220>
<223> primer

<400> 10
ctggcaaaag gcgcggtgg 19


<210> 11
<211> 17
<212> DNA
<213> Artificial

<220>
<223> primer

<400> 11
ttcctttgcg cgccact 17


<210> 12
<211> 19
<212> DNA
<213> Artificial

<220>

<223>  primer

<400>  12
cttcctttgc gcgccactc                                                    19


<210>  13
<211>  19
<212>  DNA
<213>  Artificial

<220>
<223>  primer

<400>  13
ccttcctttg cgcgccact                                                    19


<210>  14
<211>  20
<212>  DNA
<213>  Artificial

<220>
<223>  primer

<400>  14
cttcctttgc gcgccactcg                                                   20


<210>  15
<211>  16
<212>  DNA
<213>  Artificial

<220>
<223>  primer

<400>  15
cggtggggag tgccta                                                       16


<210>  16
<211>  16

<212>  DNA
<213>  Artificial

<220>
<223>  primer

<400>  16
cggtggggag tgcctc                                                        16


<210>  17
<211>  16
<212>  DNA
<213>  Artificial

<220>
<223>  primer

<400>  17
cggtggggag tgccct                                                        16